## Europäisches Patentamt

## European Patent Office

## Office européen des brevets

(11) Veröffentlichungsnummer: **0 017 999**
**B1**

(12) **EUROPÄISCHE PATENTSCHRIFT**

(45) Veröffentlichungstag der Patentschrift:
02.03.83

(51) Int. Cl.³: **A 61 B 5/14**

(21) Anmeldenummer: **80102102.3**

(22) Anmeldetag: **18.04.80**

(54) **Blutlanzette.**

(30) Priorität: **21.04.79 DE 7911762 U**

(43) Veröffentlichungstag der Anmeldung:
**29.10.80 Patentblatt 80/22**

(45) Bekanntmachung des Hinweises auf die Patenterteilung:
**02.03.83 Patentblatt 83/9**

(84) Benannte Vertragsstaaten:
**AT BE CH DE FR GB IT LI LU NL SE**

(56) Entgegenhaltungen:
**FR-A-1 188 965**
**FR-A-2 393 586**
**US-A-2 622 597**
**US-A-2 896 628**
**US-A-3 034 507**
**US-A-3 517 670**
**US-A-3 964 482**

(73) Patentinhaber: **Intermedicat GmbH, Gerliswilstrasse 45, CH-6020 Emmenbrücke (CH)**

(72) Erfinder: **Griesel, Karl-Heinz, Oberer Weinberg 19, D-3508 Melsungen 5 (DE)**

(74) Vertreter: **von Kreisler, Alek, Dipl.-Chem. et al, Deichmannhaus am Hauptbahnhof, D-5000 Köln 1 (DE)**

Blutlanzette

Die Erfindung bezieht sich auf eine Blutlanzette zur Punktion der Haut, bestehend aus einem flachen Stäbchen aus hartem Material, mit einem eine Spitze aufweisenden Einstechteil, dessen Materialstärke zur Spitze hin abnimmt.

Blutlanzetten werden in der Medizin in großer Zahl zur Punktion, z. B. der Fingerkuppe, verwendet, um einige Tropfen Blut für Blutuntersuchungen zu gewinnen. Sie sind für den Einmalgebrauch bestimmt und sollten u. a. folgende Anforderungen erfüllen: Stabiles, korrosionsbeständiges Stäbchen, das sich preiswert herstellen läßt; begrenzte Einstichtiefe der Spitze für einen schnell heilenden, schmerzarmen Einstich und einfache, gefahrlose Handhabung durch den Anwender.

Bekannte Blutlanzetten (US-A-2 896 628; 3 517 670) bestehen aus einem flachen Stäbchen aus Metall mit mindestens einer Einstechspitze. Sie werden aus rost- und säurebeständigem Stahl gestanzt, der ein hochwertiger und teurer Werkstoff ist, welcher aufgrund des hohen Cr—Ni-Gehaltes außerdem noch einen raschen Verschleiß der Stanzwerkzeuge herbeiführt. Die Materialstärke des aus Metall gestanzten Stäbchens ist entweder über seine ganze Erstreckung einschließlich des Einstechteiles gleich, so daß an der Spitze im Querschnitt gesehen vier Kanten vorhanden sind und die Spitze nicht scharf genug ist, um einen schnellheilenden schmerzarmen Einstich zu verursachen (US-A-3 517 670), oder es wird nachträglich der Einstechteil so geschmiedet, daß die Materialstärke zur Spitze hin abnimmt (US-A-2 896 628). Ein solches nachträgliches Schmieden verteuert eine bereits durch den Werkstoff kostspielige Blutlanzette aus Metall jedoch zusätzlich, und dies ist bei dem nur einmal benutzten Instrument unerwünscht. Die beiden bekannten Blutlanzetten aus Metall sind im übrigen nachteilig für den Anwender, weil dieser sich bei ihrer Handhabung an den scharfen Kanten der Längsränder und des spitzenfernen Endes leicht verletzen kann.

Ferner ist eine Vorrichtung zum Einführen von Medikamenten unter die Haut bekannt (US-A-2 622 597), die aus einem billigeren, plattenförmigen Kunststoffkörper mit einem eine Spitze aufweisenden Einstechteil besteht, dessen Materialstärke zur Spitze hin gleichbleibt. Dies verursacht beim Einstechen der Spitze in die Haut starke Schmerzen und der verhältnismäßig grobe Einstich heilt schlecht. Eine andere bekannte Vorrichtung (US-A-3 034 507) besteht ebenfalls aus einem Kunststoffkörper mit angeformten Spitzen, deren Materialstärke insgesamt gleichbleibt, so daß auch sie zur Durchführung schmerzarmer Einstiche nicht geeignet sind.

Der Erfindung liegt die Aufgabe zugrunde, eine Blutlanzette mit einem eine Spitze aufweisenden Einstechteil, dessen Materialstärke zur Spitze hin abnimmt, damit ein schnellheilender schmerzarmer Einstich durchführbar ist, so auszubilden, daß sie billig herstellbar ist und sich vom Anwender gefahrlos handhaben läßt.

Diese Aufgabe wird dadurch gelöst, daß das Stäbchen aus zähem und hartem Kunststoff besteht und neben seiner Spitze zwei messerartige Schneiden aufweist.

Eine solche Blutlanzette läßt sich preiswert als Massenprodukt herstellen, weil Kunststoff billiger ist als Stahl und weil die Blutlanzette einschließlich der messerartigen Schneiden an der Spitze des Einstechteiles in einem einzigen Arbeitsgang hergestellt werden kann. Durch entsprechende Auslegung von Stanz- und Spritzwerkzeugen können die Flanken der Spitze die gewünschte Schärfe erhalten. Außerdem kann bei der Herstellung im Spritzgußverfahren die Materialdicke des Stäbchens in jedem Bereich den Erfordernissen entsprechend frei gewählt werden, so daß das Stäbchen stabil ist und sich vom Anwender gut und gefahrlos erfassen und handhaben läßt. Beispielsweise kann für den Einstechteil eine möglichst geringe Wanddicke und für das Griffstück eine etwas größere Wanddicke vorgesehen werden. Die scharfe Spitze ermöglicht einen besonders schmerzarmen Einstich mit begrenzter Einstichtiefe, der eine ausreichende Menge von Blut abgibt und schnell heilt.

In vorteilhafter Ausgestaltung der Erfindung weist zur Verminderung der Verletzungsgefahr für den Anwender das Stäbchen eine äußere Randverstärkung und/oder am oberen Ende eine Auflagefläche für die Fingerkuppe auf. Zum Einstechen wird mit der Fingerkuppe auf den oberen Rand des Stäbchens gedrückt, wobei die vergrößerte Auflagefläche für die Fingerkuppe die Einstechkraft auf eine möglichst große Fläche verteilt, so daß der Einstich kräftig und praktisch schmerzlos erfolgt.

Das Stäbchen ist zur Versteifung mit mindestens einer längslaufenden Verstärkungsrippe versehen. Sie kann an wenigstens einer Schmalseite oder wenigstens einer Breitseitenfläche des Stäbchens angeformt sein. Bei Herstellung der Blutlanzette im Spritzgußverfahren geschieht die Anformung der Verstärkungsrippen während der Herstellung des Stäbchens. Wenn die Lanzetten aus Tafeln oder Bändern gestanzt sind, werden die Verstärkungsrippen eingepreßt.

Die Erfindung wird nachstehend anhand eines in der Zeichnung veranschaulichten Beispiels näher erläutert.

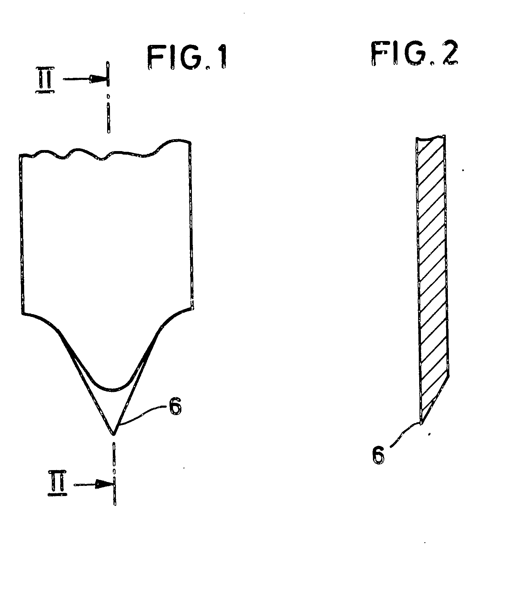

Fig. 1 zeigt eine Spitze einer Blutlanzette mit messerartigen Schneiden in Vorderansicht und

Fig. 2 ist ein Längsschnitt entlang der strichpunktierten Linie II-II in Fig. 1.

Zur Erzielung einer scharfen Spitze muß der für die Blutlanzette verwendete Kunststoff eine möglichst große Härte aufweisen. Gleichzeitig muß er eine hohe Zähigkeit besitzen, damit der Einstechteil während der Punktion nicht abbrechen kann. Kunststoffe mit dieser Kombination von Eigenschaften sind zum Beispiel Polyacetat, Polycarbonat, ABS oder PCV-hart, sowie einige mit Glasfasern, Glaskugeln o. dgl. gefüllte Kunststoffe.

Die Verwendung von Kunststoff erlaubt die Herstellung der Blutlanzetten im Spritzgußverfahren, wobei die Materialdicke in jedem Bereich des Stäbchens den Erfordernissen entsprechend frei gewählt werden kann. Außerdem können die Blutlanzetten aus Tafeln oder Bändern gestanzt werden.

Durch entsprechende Auslegung der Stanz- und Spritzwerkzeuge ist es möglich, die Spitze des Kunststoffstäbchens in einem Arbeitsgang mit messerartigen Schneiden 6 zu versehen. Die scharfe Spitze ermöglicht einen besonders schmerzarmen Einstich.

**Patentansprüche**

1. Blutlanzette zur Punktion der Haut, bestehend aus einem flachen Stäbchen aus hartem Material, mit einem eine Spitze aufweisenden Einstechteil, dessen Materialstärke zur Spitze hin abnimmt, dadurch gekennzeichnet, daß das Stäbchen aus zähem und hartem Kunststoff besteht und neben seiner Spitze zwei messerartige Schneiden (6) aufweist.

2. Blutlanzette nach Anspruch 1, dadurch gekennzeichnet, daß das Stäbchen eine äußere Randverstärkung und/oder am oberen Ende eine Auflagefläche für die Fingerkuppe aufweist.

3. Blutlanzette nach Anspruch 1 oder 2, dadurch gekennzeichnet, daß das Stäbchen mit mindestens einer längslaufenden Verstärkungsrippe versehen ist.

4. Blutlanzette nach Anspruch 3, dadurch gekennzeichnet, daß an wenigstens einer Schmalseite des Stäbchens eine Verstärkungsrippe angeformt ist.

5. Blutlanzette nach Anspruch 3, dadurch gekennzeichnet, daß die Verstärkungsrippe auf mindestens einer Breitseitenfläche des Stäbchens angeformt ist.

**Claims**

1. Blood letting lancet for piercing the skin comprising a flat little rod of hard material, a piercing portion having a point and a material thickness which decreases towards the point, characterized in that the little rod consists of tough and hard plastics and that it includes adjacent to its point two knife-type blades (6).

2. Blood letting lancet according to claim 1, characterized in that the little rod is provided with an outer reinforced edge and/or a supporting surface at its upper end for the finger tip.

3. Blood letting lancet according to claims 1 or 2, characterized in that the little rod is provided with at least one longitudinally extending reinforcing rib.

4. Blood letting lancet according to claim 3, characterized in that a reinforcing rib is integrally molded to at least one narrow side of the little rod.

5. Blood letting lancet according to claim 3, characterized in that the reinforcing rib is integrally molded to at least one broad side of the little rod

**Revendications**

1. Lancette à sang pour ponction au niveau de la peau, se composant d'une barrette plate en matériau dur, pourvue d'une partie d'enfoncement comportant une pointe et dont l'épaisseur de matière diminue en direction de la pointe, caractérisée en ce que la barrette est constituée d'une matière plastique tenace et dure et comporte à côté de la pointe deux arêtes de coupe en forme de lame de couteau (6).

2. Lancette à sang selon la revendication 1, caractérisée en ce que la barrette comporte un renforcement de bord extérieur et/ou, à l'extrémité supérieure, une surface d'appui pour le bout de doigt.

3. Lancette à sang selon l'une des revendications 1 ou 2, caractérisée en ce que la barrette est pourvue d'au moins une nervure de renforcement orientée longitudinalement.

4. Lancette à sang selon la revendication 3, caractérisée en ce qu'une nervure de renforcement est formée sur au moins un côté étroit de la barrette.

5. Lancette à sang selon la revendication 3, caractérisée en ce que la nervure de renforcement est formée sur au moins une surface latérale large de la barrette.

FIG.1

FIG.2

6

6